# EUROPEAN PATENT APPLICATION

(11) **EP 4 424 176 A1**
(43) Date of publication of application: **04.09.2024**
(21) Application number: 24160815.7
(22) Date of filing: 01.03.2024
(51) Int. Cl.: A23L 33/105, A23L 33/12, A23L 33/15, A61K 36/02, A23D 9/007, A23D 9/04, A23L 33/155

(54) **DIETARY SUPPLEMENT BASED ON OMEGA-3 ACIDS OF VEGETABLE ORIGIN AND A FORM OF VITAMIN D**

(30) Priority: 03.03.2023 EP 23159953
(71) Applicant: Enervit S.p.A., 20149 Milano (IT)
(72) Inventor: PETRONI, Paolo, 20833 GIUSSANO (IT); MUSAZZI, Diego, 20025 LEGNANO (IT)
(74) Representative: Cattaneo, Elisabetta

(57) **Abstract**

The present invention concerns a composition based on at least one omega-3 polyunsaturated fatty acids (n-3 PUFA) preferably of non-animal origin in combination with a hydroxylated form of vitamin D. The combination of the components of the composition results in a synergistic anti-inflammatory action on the human organism that makes it suitable for use in the prevention or treatment of an inflammatory disease.

## Description

### FIELD OF THE INVENTION

The invention concerns a composition and a supplement based on a combination of omega-3 acids of vegetable origin and a hydroxylated form of vitamin D. The supplement is suitable in the treatment and in the prevention of an inflammatory state of the human organism or of an inflammatory disease, in particular of a chronic type. The present invention originates in the field of dietary supplements and nutraceutical/functional products, and in particular it concerns a composition based on selected active ingredients of non-animal origin whose synergistic enhanced action allows to reduce the inflammatory state of the human organism or to treat an inflammatory disease, in particular chronic.

### BACKGROUND ART

Inflammation is a defence mechanism of the body that is activated in the presence of harmful events of an irritative, allergic or infectious type. Inflammation is an evolutionarily conserved process, characterized by the activation of cells belonging mainly to the immune system, which protect the body from bacteria, viruses and toxins by eliminating pathogens and promoting tissue repair (Netea MG et al., Nat. Immunol. 18, 826-831 (2017). The inflammatory response is activated by the body whenever it perceives a threat, and, normally, when the factor perceived as harmful has been eradicated, it starts the process of "turning off' the inflammation, defined as resolution. Usually, the inflammatory response is well regulated and tends not to cause excessive damage to the host. In this case, inflammation is defined as self-limiting and is rapidly resolved thanks to the activation of negative feedback mechanisms, through secretion of anti-inflammatory cytokines or resolving lipid mediators, through inhibition of the pro-inflammatory cascade or through the activation of regulatory cells (Kotas ME & Medzhitov R. Cell 160, 816-827 (2015). Calder PC et al. Biochim Biophys Acta 1851, 469-484 (2015).)

The body's initial response to a harmful stimulus occurs through acute inflammation and involves an increased movement of plasma and leukocytes from the blood towards the damaged tissue. Then a cascade of biochemical events that matures the inflammatory response follows: these events are a prerogative of the local vascular system, of the immune system and of several cells of the damaged tissue. Under normal conditions, the inflammation tends to resolve quickly, in a couple of hours or in a couple of days; however, in case it is prolonged excessively, the inflammation is defined as chronic and can last even for years. Regardless of the type of event that caused the inflammation to arise, the response initially comprises an increase in blood flow towards the inflamed site, followed by an increase in the permeability of the vascular wall due to a greater opening of the junctions between endothelial cells, which allows the passage of soluble mediators at the site of inflammation. Then the leukocytes migrate from the bloodstream towards the surrounding tissue, a process that is promoted by the release of chemo-attractors from the site of inflammation and by the increase in the production of adhesion molecules at the endothelium level. Finally, the leukocytes release different types of chemical mediators at the site of inflammation depending on the cell type involved, on the nature of the inflammatory stimulus, on the anatomical site, and on the stage of the inflammatory process. Among the chemical mediators involved there may be those of lipid derivation (prostaglandins, leukotrienes, endocannabinoids, platelet activation factor), peptide mediators (cytokines, chemokines), reactive oxygen species, amino acid derivatives (histamine) and enzymes (matrix proteases) (Calder PC. Nutrients 2(3), 355-374 (2010).

Acute inflammation arises primarily from a bacterial or viral infection through an interaction between cellular receptors expressed on cells of the innate immune system and conserved structures present on the membranes of pathogens, called pathogen-associated molecular patterns (PAMP). The acute response can also be activated by "sterile" agents, defined as molecular damage-associated patterns (DAMP), which are released in response to harmful stimuli of a physical, chemical or metabolic nature during stress or cell damages (Furman D et al. Nat. Med. 25, 1822-1832 (2019).

A chronic inflammatory state is instead activated by DAMP in the absence of an acute infectious or harmful event. Among the triggers of a state of systemic inflammation, there may be the presence of biological, environmental, social and psychological factors, which often inhibit the complete resolution of the acute inflammatory state, thus promoting a state of chronic inflammation of a systemic nature. The transition from an acute to a chronic inflammatory response can be followed by severe functional and structural alterations in all tissues and organs, which, by altering the normal cell physiology, can increase the risk of chronic diseases in both adult and young populations. Chronic inflammation can also alter normal immune function, increasing susceptibility to infections, tumours, and decreasing response to vaccinations (Shen-Orr SS et al. Cell Syst. 3, 374-384 (2016).

Basically, with increasing age, the markers of the chronic systemic inflammation also increase: higher levels of cytokines, chemokines and acute phase proteins are found, as well as greater expression of genes involved in inflammation. The establishment of the chronic systemic inflammation can cause over time several collateral damages to tissues and organs, leading to the development of severe pathological conditions such as metabolic syndrome, type 2 diabetes, cardiovascular diseases, different types of cancer, autoimmune and neurodegenerative diseases, consequently reducing the quality and life expectancy of people who are affected by them (Franceschi C et al. Trends Endocrinol. Metab. 28, 199-212 (2017).

Therefore, it is appropriate to evaluate the intake of long-term supplements, which, together with some targeted changes in lifestyle such as diet, physical activity, stress, can improve, both preventively and therapeutically, the inflammatory status of an individual by limiting the negative health outcomes.

The need is therefore currently felt to provide products suitable for reducing the development of the inflammation, in particular chronic inflammation, whose dispensation to the public is not subject to medical prescription and whose administration is free of side effects also because the lifestyles of modern society make inflammation of the organism in increasingly young populations more and more frequent.

### SUMMARY OF THE INVENTION

The Applicant of the present invention has observed that a specific composition comprising a combination of omega-3 polyunsaturated fatty acids (n-3 PUFA) with calcifediol, a hydroxylated form of vitamin D, is endowed with beneficial properties in the absence of side effects, in the treatment and in the prevention of an inflammatory state of the human organism or of an inflammatory disease.

Surprisingly, the Applicant found out that when the omega-3 polyunsaturated fatty acids (n-3 PUFA) comprise (eicosapentaenoic acid) EPA and (docosahexaenoic acid) DHA in a weight ratio of EPA:DHA in the range from 0.25:1 to 3:1, the composition that includes them together with the calcifediol showed better and enhanced beneficial properties in the prevention and treatment of an inflammatory state of the human organism or an inflammatory disease on a subject in need thereof, due to a synergistic effect between the components. The invention therefore concerns a composition comprising a combination of omega-3 polyunsaturated fatty acids (n-3 PUFA) with calcifediol, and a physiologically acceptable carrier, wherein said omega-3 polyunsaturated fatty acids (n-3 PUFA) comprise (eicosapentaenoic acid) EPA and (docosahexaenoic acid) DHA in a weight ratio of EPA:DHA in the range from 0.25:1 to 3:1 in the prevention and treatment of an inflammatory state of the human organism or an inflammatory disease on a subject in need thereof.

In a further aspect the invention relates to a dietary supplement comprising the composition above stated and excipients.

In a still further aspect, the invention relates to a non-therapeutical use of the composition or of the supplement of the invention for improving the inflammatory status of a subject.

The dependent claims appended herein describe embodiments of the invention.

### DETAILED DESCRIPTION OF THE INVENTION

The present invention originates from having surprisingly identified a composition comprising a combination of omega-3 polyunsaturated fatty acids (n-3 PUFA) with calcifediol, wherein said omega-3 polyunsaturated fatty acids (n-3 PUFA) comprise (eicosapentaenoic acid) EPA and (docosahexaenoic acid) DHA in a defined weight ratio of EPA:DHA and wherein said composition is endowed with beneficial properties in the absence of side effects, in the prevention and in the treatment of an inflammatory state of the human organism or of an inflammatory disease of a subject in need thereof.

According to the present invention, with the terms "subject" and/or "individual" is intended a healthy subject or a patient, which presents an inflammatory condition, disorder or disease, a symptom of the inflammatory condition, disorder or disease, or a predisposition towards a inflammatory condition, disorder or disease, for the purpose of curing, healing, alleviating, altering, remedying, make improvements in, bring benefit to, or prevent or treat the inflammatory condition, disorder or disease, symptoms of the condition, disorder or disease.

The invention, therefore, in a first aspect, concerns a composition for oral administration comprising a combination of omega-3 polyunsaturated fatty acids (n-3 PUFA) with calcifediol, and a physiologically acceptable carrier, wherein said omega-3 polyunsaturated fatty acids (n-3 PUFA) comprise (eicosapentaenoic acid) EPA and (docosahexaenoic acid) DHA in a weight ratio of EPA:DHA in the range from 0.25:1 to 3:1.

In a preferred embodiment, said weight ratio of EPA:DHA is in the range from 0.3:1 to 3:1.

In a further preferred said weight ratio of EPA:DHA is in the range from 0.33:1 to 3:1.

In an even more preferred embodiment, said omega-3 polyunsaturated fatty acids further comprise at least an omega-3 polyunsaturated fatty acid selected from the group consisting of α-linolenic acid (ALA), stearidonic acid (SDA), eicosatrienoic acid (ETE), eicosatetraenoic acid (ETA), docosapentaenoic acid (DPA), tetracosapentaenoic acid andtetracosahexaenoic acid.

In a further even more preferred embodiment, said omega-3 polyunsaturated fatty acids further comprise docosapentaenoic acid (DPA).

Omega-3 polyunsaturated fatty acids are, together with omega-6, part of the category of essential fatty acids. They are best known for their presence in cell membranes and for maintaining the integrity thereof and have very different roles in the inflammatory response. Omega-6, substances like hormones, start the inflammatory process to protect the body from different types of offences such as, for example, microbial invasions. Among omega-6s, arachidonic acid is the major precursor to a wide variety of pro-inflammatory eicosanoids. Its dosages in the blood should be between 7% and 9% of the total fatty acids, but due to a wrong diet these levels are often higher, contributing to a higher inflammatory state.

Eicosanoids are key lipid mediators of inflammation, include oxidized derivatives of 20-carbon polyunsaturated fatty acids, and include prostaglandins, thromboxanes and leukotrienes. The main precursor of the eicosanoids is the arachidonic acid, which is released from the membrane phospholipids of the inflammatory cells through the action of phospholipase A2, an enzyme that is activated by the inflammatory stimulus. Arachidonic acid acts as a substrate for the cyclo-oxygenase (COX), lipooxygenase (LOX) and cytochrome P450 enzymes, which produce pro-inflammatory mediators such as PGE2 and LTB4.

Eicosanoids act through specific receptors, such as the receptors coupled to protein G. Among the eicosanoids, mention can be made of the endocannabinoids, produced starting from phospholipids whose bonds have been cut by phospholipases other than A2.

Endocannabinoids exert anti-inflammatory action and can be produced starting from the omega-3 fatty acids EPA and DHA.

It has been also observed that, following the use of omega-3-based supplements, there is a reduced production of prostaglandins and leukotrienes from arachidonic acid.

Omega-3s make up the fundamental components of the group of hormones called resolvins, which help resolve the inflammatory process. The intake of omega-3s containing EPA and DHA tends to shift the profile of the membrane fatty acids towards a lower content of arachidonic acid, and consequently a lower production of inflammatory eicosanoids occurs. Typically, the omega-3 polyunsaturated fatty acids exert an anti-inflammatory effect by regulating the production of pro-inflammatory cytokines (TNF, IL-1b, IL-6). In particular, n-3 PUFAs, preferably EPA and DHA, are capable of inhibiting the production of TNF, IL-1b and IL-6 by macrophages. Some studies on animal models have shown an effect of the omega-3s in increasing the levels of the anti-inflammatory cytokine IL-10. Similar results were also found in human subjects, where it is assumed that the mechanism involved may be a reduction in gene expression of the pro-inflammatory cytokines, by action on the pathways NFkB and PPAR-γ.

This mechanism of regulating the inflammation leads to reduced levels of circulating pro-inflammatory cytokines. In addition, potent anti-inflammatory metabolites such as resolvins, maresins, and protectins, collectively called specialized pro-resolution mediators, are derived from omega-3 metabolism. The biological effects of resolvins, protectins and maresins have been extensively examined in cell cultures and animal models of inflammation, demonstrating an anti-inflammatory and inflammation-resolving effect. Resolvins are able to inhibit the infiltration and the transendothelial migration of neutrophils, cells belonging to the innate immune response. In addition, the resolvins inhibit the production of pro-inflammatory mediators, and have a potent anti-inflammatory and immunoregulatory effect. This leads to less activation of the inflammatory cascade, allowing the inflammation to be controlled. Pro-resolution molecules work by activating specific mechanisms that promote homoeostasis. Specifically, these molecules stimulate resolution through multifactorial mechanisms at the tissue interface. Specific resolvins and protectins provide a powerful stimulus that selectively blocks the infiltration of neutrophils and eosinophils. In addition, they stimulate the recruitment of monocytes without pro-inflammatory mediators, activate macrophage phagocytosis, increase phagocyte removal, and stimulate the expression of antimicrobial defence mechanisms. Several studies have also pointed out an effect on the resolution of the inflammation through MAPK and NF-kB cell signalling pathways, thus performing an anti-inflammatory effect based on gene transcription regulation. It has also been observed that omega-3 supplementation leads to a reduction in neutrophil and monocyte chemotaxis towards several chemo-attractors such as LTB4, bacterial peptides and human serum. Since chemotaxis is the process by which leukocytes move to the site of inflammation in response to the release of molecules called chemo-attractors, the result of this omega-3 mediated regulation mechanism is a lower presence of pro-inflammatory cells on the site of inflammation, and consequently a lower activation of the inflammatory cascade.

Studies on cell cultures and animal models have shown that omega-3s can reduce the expression of certain adhesion factors on the surface of monocytes, macrophages, lymphocytes, and endothelial cells, effectively reducing the adhesive interactions between monocytes and endothelial cells. The adhesion factors are proteins that are expressed on the surface of the endothelial cells and leukocytes. These molecules promote the interaction between different cell types: it is thanks to these interactions that the leukocytes in the blood can interact with the walls of the vessels and cross the endothelial junctions to reach the site of inflammation. In human subjects, EPA and DHA supplementation has led to lower levels of ICAM-1 adhesion factor expression on the surface of IFN-γ-stimulated monocytes.

This mechanism has a combined and additive effect with the reduction of chemotaxis: fewer leukocytes reaching the site of inflammation means less secretion of pro-inflammatory mediators and consequently greater control over the inflammatory response.

The composition of the invention as claimed allows to guarantee all the know properties of EPA.

The fatty acid EPA is in fact able to lower plasma triglyceride levels and reduce levels of pro-inflammatory cytokines and chemokines.

Specifically, the literature has shown several direct benefits of the fatty acid EPA, such as:
- Reduction of triglyceride and cholesterol levels
- Protection from oxidative damage
- Inhibition of monocyte movement and conversion into macrophages in the preliminary stage of lesions
- Support antioxidant and anti-inflammatory activities of HDL (high-density lipoprotein)
- Promotion of cholesterol efflux from macrophages
- Reduction of the formation of atherosclerotic plaques
- Reduction of platelet-mediated thrombus formation
- Reduction in blood pressure, due to improvement in endothelial function.

Furthermore, omega-3 EPA is a precursor of Resolvin E1 (RvE1), a positive mediator of inflammation capable of reducing inflammation and neutrophil migration, activating the inflammation resolution process.

RvE1 reduces the release of pro-inflammatory cytokines, mainly interleukins (IL-1, IL-6, IL-12, IL-17 and IL-23).

In addition to reducing pro-inflammatory cytokines, RvE1 inhibits inflammatory angiogenesis and stimulates phagocytosis via macrophages. This strong anti-inflammatory activity of EPA-derived resolvins E has protective effects not only on the circulatory system, but also on the respiratory system, where they facilitate apoptosis and the resolution of inflammation of the respiratory tract following contact with allergens.

Another molecule derived solely from EPA is resolvin E2 (RvE2). This molecule also has anti-inflammatory activity: RvE2 stimulates phagocytosis and regulates integrins on the surface of leukocytes, facilitating their migration to the site of inflammation, and at the same time limiting the recruitment of granulocytes. Furthermore, it promotes the production of the anti-inflammatory cytokine IL-10 by macrophages.

Finally, the fatty acid EPA also presents a distinctive activity, not found in DHA: EPA is able to modulate and reduce the activity of the Δ6-desaturase enzyme, responsible for the conversion process from EPA to DHA.

Therefore, the composition of the invention comprising EPA, in addition to DHA, in the the weight ratio in the range from 0.25:1 to 3:1 allows to maintain the specific benefits of EPA listed above, avoiding its direct conversion into DHA within the body.

Taken together, these results demonstrate how omega-3 polyunsaturated fatty acids can cause a shift from a strongly pro-inflammatory environment to a state of reduced inflammation, with lower cellular reactivity and higher resolution of inflammation.

The inventors found out that the specific weight ratio as claimed of EPA and DHA in combination with calcifediol allowed not only to guarantee the anti-inflammatory activity of EPA, but also showed a synergistic effect between the two selected omega-3 polyunsaturated fatty acids in the weight ratio in the range from 0.25:1 to 3:1 and calcifediol.

According to a preferred embodiment of the present invention, the EPA and DHA, are not of animal origin and are preferably contained in an oil of non-animal origin.

According to a more preferred embodiment of the present invention, all the omega-3 polyunsaturated fatty acids present in the composition are not of animal origin and are preferably contained in an oil of non-animal origin.

In the present invention when the term "non-animal oil" is used it is meant to comprise both vegetable and synthetic oils.

In a preferred embodiment, the oil of non-animal origin according to the present invention is obtained by extraction from a microalga, preferably *Schizochytrium sp.* Therefore, the oil of non-animal origin is preferably a vegetable oil.

In a preferred embodiment, the composition of the invention also comprises an omega-6 polyunsaturated fatty acid, arachidonic acid. Said arachidonic acid is preferably contained in an oil of non-animal origin. The arachidonic acid content in the non-animal oil is preferably less than 20 mg, more preferably it is comprised from 1 mg to 13 mg per gram of oil.

The use of an oil of non-animal origin in the composition described herein has numerous advantages. Many studies in the literature have confirmed the presence of extremely low levels of arachidonic acid in the DHA-containing oils of vegetable origin. For example, the arachidonic acid content in the analysed oils from microalgae *Schizochytrium sp.* containing DHA is between 1 mg and 13 mg per gram of oil, while in commonly commercially available fish oil the arachidonic acid levels exceed 20-25 mg per gram of oil (Yokochi T. Appl Microbiol Biotechnol 49, 72-76 (1998), Zhu L. Proc Biochem 42, 210-214 (2007), Zou X. Appl Biochem Biotech 191, 1294-1314 (2020), EFSA Journal 2020;18(10):6242, EFSA Journal 2021;19(1):6345, Dossier Novel Food Martek (2010)).

The lower arachidonic acid content of the EPA/DHA-containing oil of vegetable origin used in the context of the invention, compared to the animal source oil commonly present on the market therefore guarantees an even greater anti-inflammatory potential to the composition of the invention, thanks precisely to the lower arachidonic acid content known for its pro-inflammatory effects.

The use of omega-3s of non-animal origin also helps to solve food safety problems typical of omega-3s of animal origin. In particular, the fish allergen is completely absent in non-animal oil, and this allows for the intake of omega-3s in total safety even in individuals allergic to fish and derivatives. The vegetable origin also makes it possible to limit environmental problems related to the procurement of raw material from "non-renewable" animal sources. By limiting the human impact on the marine ecosystem, it also allows to preserve the natural ecosystem of this environment, and opens the doors to a wider production of omega-3s to allow a sustainable integration of as many people as possible. Although the omega-3 supplements obtained from high-quality fish have very low levels of marine contaminants, the microalgae grown in closed tanks or industrial fermenters are completely free of heavy metals and other contaminants typical of the marine environment. This also solves the potential problem of bioaccumulation of certain contaminants that are not metabolised and tend to accumulate over time within the body.

Advantageously, the long-chain omega-3-based oil of vegetable origin used in formulating the composition of the invention differs from a long-chain omega-3-based oil of animal origin in the different structural stereochemistry of the triglyceride molecules that make up the oil.

In addition, the vegetable oil contains triglycerides with certain fatty acids in certain preferential positions, clearly different from what can be observed in animal oil. In particular, in the vegetable oil obtained from the microalga *Schizochytrium sp.* it can be observed that the fatty acid DHA is distributed equally in all 3 positions of the triglyceride, while the central position of the triglyceride (sn-2) is preferably occupied by myristic acid (Zhang T. J Am Oil Chem Soc 93, 1337-1346 (2016). In fish oil, on the other hand, regardless of the species chosen, it has been found that the fatty acid DHA is distributed preferentially in the central position of the triglyceride (sn-2), while the fatty acid EPA is distributed equally in all 3 positions of the triglyceride (Zhang H. Biomed Res Int 3529682, 1-10 (2018), Suarez ER. J Am Oil Chem Soc 87, 1425-1433 (2010).

In addition, the oils from different species show a different triglyceride spectrum. In particular, the triglyceride profile in the vegetable-origin oil is completely different from the triglyceride profile in the oil of animal origin.

For these reasons the use of an oil of a non-animal, preferably vegetable, nature in the composition of the invention is appreciable.

The further essential component of the composition is calcifediol, a particular form of biologically active vitamin D3.

Vitamin D3 is part of a group of fat-soluble prohormones which consist of 5 different vitamins: vitamin D1, D2, D3, D4 and D5. The two most important forms in which vitamin D can be found are vitamin D2 (ergocalciferol) and vitamin D3 (cholecalciferol), both with very similar biological activity. Ergocalciferol (D2) comes from plants, while cholecalciferol (D3), derives from cholesterol and is synthesized in animal organisms.

Vitamin D3 can be produced by the human body upon sunlight exposure. However, in periods with inadequate sunlight vitamin D needs to be ingested from dietary sources to prevent vitamin D deficiency. Vitamin D deficiency has been associated with numerous diseases such as hyperparathyroidism, osteoporosis, cardiovascular and autoimmune diseases in addition to cognitive impairment.

70% of the Vitamin D available to the body originates from the action of solar radiation and 30% is of food origin.

Therefore, the main source of vitamin D for the human body is exposure to solar radiation.

In fact, cholecalciferol (D3) is produced by the irradiation of 7-dehydrocholesterol while ergocalciferol (D2) is formed when ultraviolet rays hit ergosterol, its pro-vitamin form of plant origin.

Vitamin D obtained from sun exposure or through diet is present in a biologically inactive form and must undergo two hydroxylation reactions to be transformed into calcitriol, the biologically active form.

Activated Vitamin D or calcitriol (1,25-dihydroxy-vitamin D) originates from the transformation of 7-dehydrocholesterol (7-DHC) on the skin according to various stages. First the irradiation leads to the formation of pre-VIT D3 and then to the formation of cholecalciferol or Vitamin D3; Ergosterol (Vit D2) is of exclusively vegetal production and therefore ingestible by animals only through food. These are prohormones and therefore have no biological activity, requiring two essential steps.

Cholecalciferol or Vitamin D3 is metabolised in the body into its more active metabolites. In fact, cholecalciferol or Vitamin D3 (the prevalent and most important vitamin) with its binding protein reaches the liver where the first activation process takes place, acquiring hydroxylation to 25-hydroxyvitamin D3 (also known as 25-hydroxycalciferol) or calcifediol. The second process occurs at the level of the renal tubules with a second hydroxylation to calcitriol (1,25-dihydroxy-vitamin D), a metabolically active form of the Vit D hormone.

Therefore, it is necessary to distinguish:
- cholecalciferol (Vitamin D3), metabolically inactive;
- calcifediol (25-hydroxyvitamin D3, also known as 25-hydroxycalciferol), a semi-activated form at the liver level and the more effective form of vitamin D in reaching optimal levels;
- calcitriol, (1,25-dihydroxy-vitamin D) active double hydroxylation form.

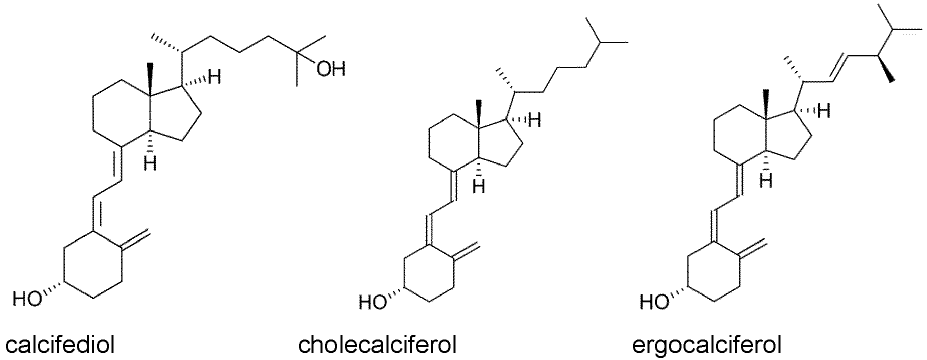

The active form of vitamin D (1,25-dihydroxy-vitamin D) has a potent anti-inflammatory action due to a switch from the phenotype of T *helper* immune cells, Th1/Th17, of pro-inflammatory nature, to the mainly anti-inflammatory Th2/Treg phenotype. The T *helper* Th1 and Th17 cells, which intervene in the adaptive immune response, have a strongly pro-inflammatory action profile, aimed at combating the pathogens through the secretion of pro-inflammatory cytokines such as IFN-γ, TGF-b, IL-6. In contrast, the response of Th2 and Treg cells is mainly anti-inflammatory, thanks to the secretion of anti-inflammatory cytokines such as IL-4, IL-10 and IL-2, and to the ability, typical of Treg cells, to maintain an immune homoeostasis avoiding phenomena of self-reactivity (Krajewska M. Front. Endocrinol. 13:920340 (2022).

Vitamin D is able to impair the development of Th17 cells starting from the undifferentiated precursor CD4, while increasing at the same time the development of regulatory cells capable of secreting IL-10. Inhibition occurs through the activation of the transcription factor VDR, which turns off the gene expression of RORγt thus inhibiting the development of Th17 cells (Palmer MT. J. Biol. Chem. 286(2), 997-1004 (2011).

This change in the profile of the T cell population leads to a decreased secretion of pro-inflammatory mediators, such as IFN-γ, TNF-a, IL-1b, IL-6, IL-8, IL-12, IL-17 and an increase in the production of anti-inflammatory cytokines such as IL-4 and IL-10.

In addition, it has been observed that vitamin D protects stable levels of the anti-inflammatory cytokine IL-10.

Several in vitro studies and studies on animal model have also pointed out a potent anti-inflammatory effect of vitamin D in its activated form. The overall effect is, as already reported above, the transition from a pro-inflammatory state to a state with lower inflammatory potential (Guillot X. Joint Bone Spine 77, 552-557; 2010).

For these reasons, vitamin D supplementation has long been known and used in the world of supplements and functional foods. One of the problems encountered is that it is not always possible to correct a deficiency in a short time, especially in certain environmental conditions, for example because of a limited sun exposure. In terms of diet, few foods are a suitable source of natural vitamin D, especially in the context of some dietary choices such as typical Western or vegan diets.

The vitamin D supplements available on the market provide cholecalciferol, which however can take even months to reach the levels necessary to correct the most significant deficiencies.

Most vitamin D supplements of vegetable origin available on the market instead provide ergocalciferol, whose effectiveness has been described in the literature as further and significantly inferior to that of cholecalciferol. These factors lead to two important problems. On the one hand, in the absence of supplementation, vitamin D intake is often insufficient to meet the body's needs under certain environmental conditions. On the other hand, even in the presence of supplementation, it has been observed that normal cholecalciferol-based vitamin D supplements can even take several months to reach optimal circulating levels of vitamin D.

Hence the need for a more effective form of vitamin D in reaching optimal levels. This precursor, defined as calcifediol, originates during the *in vivo* activation of vitamin D in the liver. Subsequently, calcifediol is further processed in the kidneys giving rise to the biologically active form of vitamin D, 1,25-dihydroxy-vitamin D. The calcifediol used in the invention is also of non-animal origin.

Advantageously calcifediol is three times more potent than cholecalciferol and is also able to reach the bloodstream three times faster than cholecalciferol. This would allow not only to obtain optimal levels of vitamin D with lower dosages, but also to reach these levels in a much shorter time.

The composition according to the present invention combines omega-3 polyunsaturated fatty acids (n-3 PUFA) comprising (eicosapentaenoic acid) EPA and (docosahexaenoic acid) DHA in a weight ratio of EPA:DHA in the range from 0.25:1 to 3:1, preferably in the range from 0.3:1 to 3:1, more preferably in the range from 0.33:1 to 3:1, with calcifediol, and allows to provide a synergistic enhanced anti-inflammatory effect making it suitable for use in the treatment and in the prevention of an inflammatory state of the human organism or of an inflammatory disease, preferably systemic chronic inflammation.

In a preferred embodiment, the composition of the present invention comprises an oil of non-animal origin having an overall EPA and DHA content in the range from 250 mg to 3000 mg, and calcifediol in the range from 0.83 µg to 16.7 µg.

The composition of the invention may take a wide variety of preparation forms. For example, the composition of the invention may be in solid form. In the case of preparations in solid form such as, for example, soft-gel or hard-gel capsule, bar, gummy candy, powder to be dissolved in water or ready-to-drink liquid emulsion.

Preparations in solid form may comprise one or more additives of a technological nature such as, for example, strength agents (e.g. glycerol), thickeners (e.g. carrageenan), acidity regulator (e.g. sodium hydrogen carbonate), tocopherol, structural agents (e.g. hydroxypropyl methyl cellulose, pectin, starches), gellan, lecithins, silicon dioxide, glycerol monostearate, polysorbate 80, flavourings, acidifiers (e.g. citric acid), acidity regulators (e.g. sodium citrate), fruit juice from concentrate, high oleic sunflower oil, rosemary extract, ascorbyl palmitate, dyes and/or fruit and vegetable concentrates, coating agents (e.g. carnauba wax), humectants (e.g. sorbitol), sweeteners (e.g. sucralose, stevia, erythritol).

According to some aspects of the invention the composition may be formulated as a dietary supplement.

The supplement therefore in addition to the composition of the invention also comprises suitable excipients for the final formulation.

The supplement according to the present invention may take a wide variety of forms depending on the form of preparation suitable for oral administration e.g. tablets, capsules, powders. One or more of the usual carriers used in the nutritional field and one or more excipients may be employed in the preparation of the compositions for the oral administration form.

In a further aspect the invention relates to a non-therapeutic use of the composition or of the supplement of the invention for improving the inflammatory status of a subject.

In a preferred embodiment the composition or the supplement according to the present invention is for use in the prevention and in the treatment of an inflammatory state of the human organism or of an inflammatory disease in a subject in need thereof, preferably the inflammatory state or the inflammatory disease is chronic.

### EXPERIMENTAL PART

### Example 1: composition of the invention and of a supplement containing it

For the preparation of the composition of the invention and therefore of the final product, such as for example a supplement, the following ingredients were employed:
- Oil of vegetable origin extracted from microalgae belonging to the species *Schizochytrium sp.* based on long-chain omega-3 containing EPA and DHA in a 1:2 ratio, in amounts of 525 mg per dose, such as to provide 250 mg of EPA + DHA per dose, plus the following excipients of a technological nature: high oleic sunflower oil, rosemary extract, tocopherols, ascorbyl palmitate.
- Calcifediol of non-animal origin, whose content per dose is equal to 4.15 µg, equivalent to 500 IU of "standard" vitamin D.
- Capsule, consisting of structural elements and additives of vegetable or non-animal origin in varying quantities: maize starch, strength agent: glycerol, thickener: carrageenan, acidity regulator: sodium hydrogen carbonate, tocopherol.

### Example 2: preparation of a soft-gel capsule comprising the composition according to the invention

The production process for this soft-gel format was articulated as follows:
- Extraction of omega-3-rich algal oil from microalgae, in this case from algae of the species *Schizochytrium sp.*
- Purification of the algal oil: the extracted oil was purified to remove impurities and contaminants, such as heavy metals and toxins.
- Omega-3 concentration: the purified algal oil was concentrated to increase the content of omega-3 fatty acids.
- Mixing with other ingredients: other ingredients, such as antioxidants and stabilizers, to improve the shelf-life of the product, and calcifediol of non-animal origin were added to the purified and concentrated oil.
- Encapsulation: the mixture containing the omega-3-rich algal oil and calcifediol was placed inside soft-gel capsules of non-animal origin respecting the amounts indicated in example 1.
- Drying and packaging: the capsules were dried to remove any residual moisture and are then packaged for final distribution.

The soft gel capsules so produced were then tested for their activity in the improvement of healthy status of individuals, that showed an altered inflammatory state. The individuals were orally administered with a posology and treatment duration depending on the severity of the inflammatory state. All the individuals resulted with an improved healthy status.

## Claims

1. A composition comprising a combination of omega-3 polyunsaturated fatty acids (n-3 PUFA) with calcifediol, and a physiologically acceptable carrier, wherein said omega-3 polyunsaturated fatty acids (n-3 PUFA) comprise (eicosapentaenoic acid) EPA and (docosahexaenoic acid) DHA in a weight ratio of EPA:DHA in the range from 0.25:1 to 3:1.

2. The composition according to claim 1, wherein said omega-3 polyunsaturated fatty acids (n-3 PUFA) comprise (eicosapentaenoic acid) EPA and (docosahexaenoic acid) DHA in a weight ratio of EPA:DHA in the range from 0.3:1 to 3:1, preferably in the range from 0.33:1 to 3:1.

3. The composition according to claim 1 and 2, wherein said an omega-3 polyunsaturated fatty acids comprise at least one further omega-3 polyunsaturated fatty acid selected from the group consisting of α-linolenic acid (ALA), stearidonic acid (SDA), eicosatrienoic acid (ETE), eicosatetraenoic acid (ETA), docosapentaenoic acid (DPA), tetracosapentaenoic acid and tetracosahexaenoic acid.

4. The composition according to claim 3, wherein said at least one further omega-3 polyunsaturated fatty acid is docosapentaenoic acid (DPA).

5. The composition according to anyone of claims 1-4, wherein said omega-3 polyunsaturated fatty acids are not of animal origin.

6. The composition according to anyone of claims 1-5, wherein said omega-3 polyunsaturated fatty acids are contained in an oil of non-animal origin.

7. The composition according to claim 6, wherein the oil of non-animal origin is obtained from a microalga, preferably *Schizochytrium sp.*

8. The composition according to anyone of claims 1 to 7, wherein said composition also comprises arachidonic acid, preferably contained in a non-animal oil in an amount of less than 20 mg, more preferably in an amount in the range from 1 mg to 13 mg per gram of non-animal oil.

9. A dietary supplement comprising the composition according to any one of claims 1-8, and excipients.

10. A non-therapeutical use of the composition according to anyone of 1-8 or of the supplement according to claim 9 for improving the inflammatory status of an individual.

11. A composition according to anyone of claims 1-8 or a dietary supplement according to claim 9, for use in the treatment or in the prevention of an inflammatory state or of an inflammatory disease of the human organism.

12. The composition for use or the dietary supplement for use according to claim 11, wherein the inflammatory state or inflammatory disease is chronic.

13. The composition for use or the dietary supplement for use according to anyone of claims 12-11, wherein said composition or said dietary supplement is administered orally.

14. The composition for use or the dietary supplement for use according to anyone of claims 11-13, wherein said composition or said dietary supplement is in the form of soft-gel or hard-gel capsule, bar, gummy candy, powder to be dissolved in water or ready-to-drink liquid emulsion.

15. The composition for use or the dietary supplement for use according to claim 14, wherein said composition or said dietary supplement is in the form of a soft-gel capsule.
